(19) Europäisches Patentamt
European Patent Office
Office européen des brevets

(11) **EP 0 545 147 B1**

(12) **EUROPÄISCHE PATENTSCHRIFT**

(45) Veröffentlichungstag und Bekanntmachung des Hinweises auf die Patenterteilung:
**17.03.1999 Patentblatt 1999/11**

(51) Int. Cl.⁶: **A61K 7/06**

(21) Anmeldenummer: **92119684.6**

(22) Anmeldetag: **19.11.1992**

(54) **Verwendung einer Superoxiddismutase zur Verhinderung oder Verzögerung des Ergrauens von menschlichen Haaren**

Use of super oxid dismutase for preventing or delaying human hair greying

Utilisation de superoxyde dismutase pour empêcher ou retarder le grisage des cheveux humains

(84) Benannte Vertragsstaaten:
**DE ES FR GB IT**

(30) Priorität: **04.12.1991 DE 4139921**

(43) Veröffentlichungstag der Anmeldung:
**09.06.1993 Patentblatt 1993/23**

(73) Patentinhaber:
• **Emerit, Marc**
  **77570 Chateau-Landon (FR)**
• **Palleau Production S.A.R.L.**
  **77570 Chateau-Landon (FR)**

(72) Erfinder: **Der Erfinder hat auf seine Nennung verzichtet.**

(74) Vertreter: **KEIL & SCHAAFHAUSEN Patentanwälte,**
**Cronstettenstrasse 66**
**60322 Frankfurt am Main (DE)**

(56) Entgegenhaltungen:
**EP-A- 0 124 393        EP-A- 0 327 263**
**EP-A- 0 327 345        DE-A- 2 545 986**

• **CHEMICAL ABSTRACTS, vol. 69, no. 26, 23.Dezember 1968 Columbus, Ohio, US; abstract no. 109753, ASLAN, ANA ET AL 'Hair lotion.' & RO-A-50 627 (ROMANIA, MINISTRY OF THE FOOD INDUSTRY)**
• **DATABASE WPI Derwent Publications Ltd., London, GB; AN 89-345129 [47] & JP-A-01 258 623 (Y. TANAKA)**
• **PATENT ABSTRACTS OF JAPAN vol. 013 no. 579 (C-668) ,20.Dezember 1989 & JP-A-01 242509 (ICHIMARU PHARCOS CO LTD) 27.September 1989,**
• **DATABASE WPI Derwent Publications Ltd., London, GB; AN 92-189207 [23] & JP-A-04 124 122 (SHISEIDO CO. LTD.)**

**Beschreibung**

Gegenstand der vorliegenden Erfindung ist die Verwendung mindestens eines Radikalfängers und/oder mindestens einer zur Deaktivierung nicht-radikalischer, reaktiver Sauerstoffspezies geeigneten Substanz in einer geeigneten Zubereitung zur Verhinderung oder Verzögerung des Ergrauens von Haaren.

Die natürliche Eigenfarbe des Haares wird durch eingelagerte Melaninpigmente hervorgerufen. Die mit fortschreitendem Alter beim gesunden Menschen beginnende verminderte oder ausbleibende Nachbildung dieser Melaninpigmente verursacht einen Farbverlust der Haare, der als Ergrauen der Haare sichtbar wird.

Aus der EP-OS 0 435 180 ist ein Haarbehandlungsmittel mit einem Gehalt an einem bestimmten Derivat des cyclischen Adenosinmonophosphats (cAMP) bekannt, das das Ergrauen menschlicher Haare verhindern und die Wiederherstellung der natürlichen Farbe bereits ergrauter Haare bewirken soll.

Es war bisher jedoch kein Wirkstoff bekannt, der die Einwirkung von Radikalen und/oder nicht-radikalischen reaktiven Sauerstoffspezies auf die Melaninbildung und damit das Ergrauen des menschlichen Haares verhindern oder verzögern kann.

Die Erfinder haben sich die Aufgabe gestellt, einen Wirkstoff zur Verhinderung oder Verzögerung des Ergrauens von menschlichen Haaren zur Verfügung zu stellen, der Radikale und/oder nicht-radikalische, reaktive Sauerstoffspezies deaktivieren kann.

Es wurde nun überraschender Weise gefunden, daß die Verwendung mindestens eines physiologisch verträglichen Radikalfängers und/oder mindestens einer zur Deaktivierung nicht-radikalischer reaktiver Sauerstoffspezies, beispielsweise zur Deaktivierung von Singulett-Sauerstoff, geeigneten physiologisch verträglichen Substanz in einer physiologisch verträglichen Zubereitung zur Behandlung der Kopfhaut und der Haare die gestellte Aufgabe in hervorragender Weise löst.

Unter einem Radikalfänger wird in dieser Anmeldung eine Substanz verstanden, die reaktive Radikale durch chemische Reaktion unschädlich machen kann. Radikalfänger im Sinne dieser Anmeldung können anorganische oder organische Verbindungen sowie Proteine und Enzyme oder deren Gemisch sein.

Gegenstand der vorliegenden Anmeldung ist daher die Verwendung mindestens eines physiologisch verträglichen Radikalfängers und/oder mindestens einer zur Deaktivierung nicht-radikalischer, reaktiver Sauerstoffspezies geeigneten, physiologisch verträglichen Substanz in einer physiologisch verträglichen Zubereitung zur Behandlung der Kopfhaut und der Haare zur Verhinderung oder Verzögerung des Ergrauens menschlicher Haare.

Der für die erfindungsgemäße Verwendung geeignete physiologisch verträgliche Radikalfänger und die zur Deaktivierung nicht-radikalischer, reaktiver Sauerstoffspezies geeignete physiologisch verträgliche Substanz sind bevorzugt ausgewählt aus Antioxidantien oder Metallproteiden.

Von den Antioxidantien, die für die erfindungsgemäße Verwendung geeignet sind, seien beispielsweise die folgenden genannt: Ascorbinsäure und deren $C_8$- bis $C_{18}$-Fettsäureester, beispielsweise Ascorbylpalmitat; Isoascorbinsäure und deren $C_8$- bis $C_{18}$-Fettsäureester; Gallussäure und deren $C_2$- bis $C_{22}$-Alkylester; Thiolverbindungen, beispielsweise Glutathion, N-Acetylcystein und Cystein; $\alpha$-Liponsäure; Bilirubin; Carotinoide, beispielsweise $\beta$-Carotin; Harnsäure; Alkylphenole, wie zum Beispiel Butylhydroxyanisol und Butylhydroxytoluol; Tocopherol; 2-Carboxy-2,5,7,8-tetramethyl-6-chromanol; Mannitol; Ubichinone und Ubichinole; Flavonoide oder flavonoidhaltige Pflanzenextrakte, wie beispielsweise Gynko Biloba-Extrakte, Silymarin oder Silybum marianum-Extrakte und anorganische Sulfite, wie zum Beispiel Natriumsulfit.

Von den für die Verwendung zur Verhinderung oder Verzögerung des Ergrauens geeigneten Metallproteiden seien beispielsweise Superoxiddismutase, Glutathionperoxidase und Cytochrome c genannt.

Für die erfindungsgemäße Verwendung ist Superoxiddismutase das bevorzugte Metallproteid.

Für die erfindungsgemäße Verwendung geeignet sind auch physiologisch verträgliche metallorganische Verbindungen oder Komplexe, die Superoxiddismutase-Aktivität zeigen, das heißt physiologisch verträgliche metallorganische Verbindungen oder Komplexe, welche die Disproportionierung von Superoxidionen ($O_2^-$) zu Wasserstoffperoxid und Sauerstoff katalysieren können. Einige dieser, auch als SOD mimics bezeichneten, Verbindungen oder Komplexe werden beispielsweise bei G. Czapski, S. Goldstein, Antioxidants in Therapy and Preventive Medicine, Plenum Press, New York, (1990), Seiten 45 - 50 beschrieben.

Superoxiddismutase ist als Bestandteil kosmetischer Mittel aus der DE-PS 25 45 986 bekannt. Die dort beschriebenen superoxiddismutasehaltigen Mittel sollen zur Haar- und Hautpflege, zum Schutz von Haar und Haut vor UV-Strahlen, sowie als Oxidationsschutz während der Konservierung und der Verwendung kosmetischer Mittel eingesetzt werden.

Der DE-PS 25 45 986 ist kein Hinweis auf die Verwendung von Superoxiddismutase, anderer Radikalfänger oder zur Deaktivierung nicht-radikalischer, reaktiver Sauerstoffspezies geeigneter Substanzen zur Verhinderung oder Verzögerung des Ergrauens von Haaren zu entnehmen.

Für die erfindungsgemäße Verwendung sind Superoxiddismutasen verschiedener biologischer Quellen geeignet, beispielsweise Superoxiddismutasen, die aus Bakterien, Pilzen oder Blut gewonnen werden. Für die erfindungsge-

2

mäße Verwendung sind natürliche oder durch Genmanipulation erzeugte Superoxiddismutasen geeignet.

JP-1242509 offenbart die Verwendung von Superoxiddismutasen pflanzlicher Herkunft zur Vorbengung des ergrauens von Haar.

Bevorzugt wird für die Verhinderung oder Verzögerung des Ergrauens von Haaren jedoch Superoxiddismutase, die aus Blut gewonnen wurde, verwendet. Besonders bevorzugt ist für die erfindungsgemäße Verwendung die aus Rindererythrocyten gewonnene Superoxiddismutase. Die für die erfindungsgemäße Verwendung geeignete Superoxiddismutase aus Rindererythrocyten weist besonders bevorzugt eine spezifische Aktivität von 3000 bis 3200 Einheiten/mg Protein auf.

Die Messung der spezifischen Aktivität der erfindungsgemäß verwendbaren Superoxiddismutasen erfolgte nach dem von J. M. McCord, I. Friedovich, J. Biol. Chem (1969) 244, Seite 6049 bis 6055 beschriebenen Methode, bei der in einem gekoppelten enzymatischen Test die Menge des durch Superoxidionen ($O_2$-) reduzierten Cytochrome c durch Extinktionsmessungen bei 550 nm bestimmt wird.

Eine nach diesem Test bestimmte Einheit der spezifischen Aktivität entspricht dann der Menge Superoxiddismutase, die benötigt wird, um die Bildungsrate des reduzierten Cytochrome c unter Versuchsbedingungen auf 50 Prozent zu reduzieren.

Gemäß der vorliegenden Erfindung soll zur Verhinderung oder Verzögerung des Ergrauens menschlicher Haare eine physiologisch verträgliche, insbesondere kosmetische, Zubereitung auf wäßriger, wäßrig-alkoholischer oder alkoholischer Basis Verwendung finden.

Es handelt sich dabei um Zubereitungen, die je nach ihrem sonstigen Anwendungszweck, für kürzere oder längere Zeit auf dem Haar und der Kopfhaut verbleiben und üblicherweise unter sanfter Massage der Kopfhaut mit den Händen angewandt werden.

Bevorzugt handelt es sich bei der für die erfindungsgemäße Verwendung geeigneten physiologisch verträglichen Zubereitung um ein Shampoo, eine Haarkur oder eine Haarspülung und besonders bevorzugt um ein Haarwasser.

Es ist jedoch ebenso möglich, Zubereitungen herzustellen, die hauptsächlich oder ausschließlich dem Ziel der Verhinderung oder Verzögerung des Ergrauens menschlicher Haare dienen.

Durch ihren Gehalt an mindestens einem Radikalfänger und/oder mindestens einer zur Deaktivierung nicht-radikalischer, reaktiver Sauerstoffspezies geeigneten Substanz wird bei wiederholter, regelmäßiger Anwendung der Zubereitungen gleichzeitig das Ergrauen der Haare verhindert oder verzögert. Für eine optimale Wirkung der Zubereitung ist die Aufrechterhaltung einer spezifischen Radikalfängerkonzentration beziehungweise einer spezifischen Konzentration der zur Deaktivierung nicht-radikalischer, reaktiver Sauerstoffspezies geeigneten Substanz an der Kopfhaut erforderlich, die durch regelmäßige Anwendung der Zubereitungen mindestens einmal wöchentlich erreicht wird.

Die für die erfindungsgemäße Verwendung zur Verhinderung oder Verzögerung des Ergrauens menschlicher Haare geeignete Zubereitung zur Behandlung der Kopfhaut und der Haare enthält mindestens einen Radikalfänger und/oder mindestens eine zur Deaktivierung nicht-radikalischer, reaktiver Sauerstoffspezies geeignete, physiologisch verträgliche Substanz, die vorzugsweise ausgewählt sind aus Antioxidantien und/oder Metallproteiden, insbesondere in einer Gesamtmenge von 0,01 bis 10 Gewichtsprozent.

In einer für die erfindungsgemäße Verwendung geeigneten Zubereitung zur Behandlung der Kopfhaut und der Haare, welche mindestens ein Antioxidans enthält und nach der Anwendung nicht wieder ausgespült wird, wie zum Beispiel Haarwasser, ist dieses Antioxidans in einer Menge von 0,01 bis 5 Gewichtsprozent, vorzugsweise 0,1 bis 2,5 Gewichtsprozent enthalten.

In einer für die erfindungsgemäße Verwendung geeigneten Zubereitung, die nach ihrer Anwendung wieder ausgespült wird und mindestens ein Antioxidans enthält, wie beispielsweise Haarshampoos und Haarspülungen, ist dieses Antioxidans in einer Menge von 0,5 bis 10 Gewichtsprozent, bevorzugt 3 bis 5 Gewichtsprozent, enthalten.

Eine Zubereitung zur Verhinderung oder Verzögerung des Ergrauens, welche nach der Anwendung nicht wieder ausgespült wird und mindestens ein Metallproteid enthält, soll dieses Metallproteid in einer Menge von 0,01 bis 0,5 Gewichtsprozent, vorzugsweise 0,01 bis 0,1 Gewichtsprozent, enthalten.

In einer für die erfindungsgemäße Verwendung geeigneten Zubereitung, die nach der Anwendung wieder ausgespült wird und mindestens ein Metallproteid enthält, ist dieses Metallproteid in einer Menge von 0,2 bis 1 Gewichtsprozent, vorzugsweise 0,2 bis 0,5 Gewichtsprozent, enthalten.

Die Zusammensetzung dieser Zubereitungen stellt eine Mischung aus mindestens einem Radikalfänger und/oder mindestens einer zur Deaktivierung nicht-radikalischer, reaktiver Sauerstoffspezies geeigneten Substanz mit den für solche Zubereitungen zur Behandlung der Kopfhaut und der Haare üblichen Bestandteilen, wie zum Beispiel Träger und Zusatzstoffen dar.

Der Trägerstoff kann ein für physiologisch verträgliche Zusammensetzungen üblicher Trägerstoff, wie eine Salbengrundlage, oder vor allem ein flüssiger Trägerstoff, wie zum Beispiel Wasser, wäßrig-alkoholische Mischungen oder Alkohol sein.

Hierfür geeignete Alkohole sind beispielsweise Ethanol, Propanol-1, Propanol-2 oder auch mehrwertige Alkohole wie Glycerin und Propylenglykol.

Als übliche Zusatzstoffe in den kosmetischen Zubereitungen kommen beispielsweise kosmetische Harze; Emulgatoren; anionische, kationische, nicht-ionische oder amphotere Tenside; Verdicker, wie zum Beispiel höhere Fettalkohole, Stärke; Cellulosederivate, Paraffinöl; ferner Pflegestoffe, wie beispielsweise Lanolinderivate, Cholesterin oder Pantothensäure; sowie weiterhin Farbstoffe; Parfümöle; Treibgase und andere in Betracht.

Die nachstehenden Beispiele sollen den Gegenstand der Erfindung näher erläutern.

**Beispiele**

| Haarshampoo | |
|---|---|
| 7,000 g | Natriumlauryethersulfat |
| 1,500 g | Kokosamidopropylbetain |
| 2,000 g | Methylglucosedioleat mit 120 Molen Ethylenoxid ethoxyliert |
| 0,400 g | Superoxiddismutase aus Rindererythrocyten einer spezifischen Aktivität von 3000 Einheiten/mg Protein |
| 5,000 g | Proteinhydrolysat |
| 0,300 g | Natriumbenzoat |
| 0,150 g | Natriumformiat |
| 0,326 g | Zitronensäure |
| 0,200 g | Natriumhydroxid |
| 0,300 g | Parfüm |
| 82,824 g | Wasser |
| 100,000 g | |

**Haarkur**

| | |
|---|---|
| 6,00 g | Glycerylstearat |
| 2,00 g | eines Gemischs aus mit 25 Mol Ethylenoxid ethoxyliertem Lauryl-, Cetyl-, Stearyl- und Oleylalkohol |
| 2,00 g | Cetylalkohol |
| 2,00 g | eines Gemischs aus Mineralöl und Lanolinalkoholen |
| 0,75 g | Quaternium-52 der Formel |

$$\left[ CH_3(CH_2)_{15-17} - N \begin{array}{c} (CH_2CH_2O)_xH \\ | \\ - (CH_2CH_2O)_yH \\ | \\ (CH_2CH_2O)_zH \end{array} \right]^+ H_2PO_4^- \quad ,$$

wobei x + y + z einen Mittelwert von 10 aufweist

| | |
|---|---|
| 0,40 g | Hydroxyethylcellulose |
| 0,40 g | Superoxiddismutase aus Rindererythrocyten einer spezifischen Aktivität von 3000 Einheiten/mg Protein |
| 0,10 g | Zitronensäure |
| 0,30 g | Salicylsäure |
| 86,05 g | Wasser |
| --------- | |
| 100,00 g | |

| Haarspülung | |
|---|---|
| 5,00 g | Cetylalkohol |
| 3,00 g | Mineralöl |
| 1,50 g | Kokosamidopropylbetain |
| 1,25 g | Cetyltrimethylammoniumchlorid |

(fortgesetzt)

| Haarspülung | |
|---|---|
| 0,40 g | Superoxiddismutase aus Rindererythrocyten einer spezifischen Aktivität von 3000 Einheiten/mg Protein |
| 0,25 g | Methylbromoglutaronitril |
| 1,00 g | Zitronensäure |
| 87,60 g | Wasser |
| 100,00 g | |

Das Haarschampoo, die Haarkur und die Haarspülung werden in der für derartige Mittel üblichen Weise und Menge verwendet und anschließend wieder aus dem Haar gespült.

| Sprühhaarkur | |
|---|---|
| 1,00 g | Oleamidopropyldimethylamin |
| 0,05 g | Superoxiddismutase aus Rindererythrocyten einer spezifischen Aktivität von 3000 Einheiten/mg Protein |
| 10,00 g | Propylenglykol |
| 10,00 g | Glycerin |
| 0,15 g | Methylparaben |
| 0,05 g | Propylparaben |
| 78,75 g | Wasser |
| 100,00 g | |

| Haarwasser | |
|---|---|
| 0,050 g | Superoxiddismutase aus Rindererythrocyten einer spezifischen Aktivität von 3000 Einheiten/mg Protein |
| 0,100 g | Allantoin |
| 0,010 g | Campher |
| 0,200 g | D,L-Panthenol |
| 0,150 g | Parfüm |
| 0,015 g | Polyvinylpyrrolidon |
| 57,600 g | Ethanol |
| 41,875 g | Wasser |
| 100,000 g | |

**Haarwasser**

| | |
|---|---|
| 1,00 g | Tocopherol |
| 1,00 g | Glycerin |
| 0,10 g | Cetylstearyltrimethylammoniumchlorid |
| 0,20 g | Parfüm |
| 40,00 g | Ethanol |
| 57,70 g | Wasser |
| 100,00 g | |

Die Sprühhaarkur und die Haarwässer werden in der für derartige Mittel üblichen Weise und Menge verwendet und verbleiben nach der Anwendung auf dem Haar und der Kopfhaut.

**Modell zur Untersuchung des Ergrauens von Haaren an schwarzen Neuseelandmäusen**

Um Versuche zur Verhinderung des Ergrauens von Haaren durchführen zu können, bedurfte es eines Untersuchungsmodells, das den Ergrauungsvorgang zuverlässig innerhalb eines gegenüber dem natürlichen Zeitablauf stark verkürzten Zeitraums ermöglicht. Ein derartiges Versuchsmodell ist eine nach Anwendung der Photosensibilisatoren 8-Methoxypsoralen oder 5-Methoxypsoralen durchgeführte ultraviolette Bestrahlung von epilierten Hautoberflächen der schwarzen Neuseelandmäuse.

Bei zwanzig schwarzen Neuseelandmäusen, die bei Versuchsbeginn zwischen 3 und 12 Monaten alt waren, wurde auf dem Rücken eine Fläche von 6 cm x 2,5 cm epiliert.

Auf die epilierte Hautoberfläche der ersten zehn schwarzen Neuseelandmäuse wurden 48 Stunden nach dem Epilieren je 50 Mikroliter der folgenden wäßrig-alkoholischen Lösung aufgetragen

| | |
|---|---|
| 0,050 g | 8-Methoxypsoralen |
| 69,965 g | Ethanol |
| 29,985 g | Wasser |
| 100,000 g | |

und kurz einmassiert.

Auf die epilierte Hautoberfläche der zweiten, ebenfalls aus zehn schwarzen Neuseelandmäusen bestehenden, Versuchstiergruppe wurden 48 Stunden nach dem Epilieren je 50 Mikroliter der folgenden wäßrig-alkoholischen Lösung aufgetragen

| | |
|---|---|
| 0,050 g | 5-Methoxypsoralen |
| 69,965 g | Ethanol |
| 29,985 g | Wasser |
| 100,000 g | |

und kurz einmassiert.

20 Minuten nach dem Auftragen der 5-Methoxypsoralenlösung beziehungsweise 8-Methoxypsoralenlösung wurden die epilierten Hautoberflächen aller zwanzig Mäuse 20 Minuten lang bei einer Wellenlänge von 365 nm mit ultraviolettem Licht bestrahlt, wobei der Abstand der Mäuse zur Ultraviolettlampe 20 cm betrug.

Für die Bestrahlung wurde eine Black Ray Longwave Ultraviolettlampe, Model B 100 A der Firma Ultraviolet Prod. Inc., San Gabriel CA, USA, die im Abstand von 20 cm eine Leistung von 3,3 bis 3,6 mW/cm$^2$ aufweist, verwendet. Die maximale Strahlungsdosis für die Mäuse betrug 4 J/cm$^2$.

Die auf der vor der Bestrahlung epilierten Hautoberfläche des Rückens der Mäuse nachwachsenden Haare waren bei allen Versuchstieren, im Gegensatz zu den auf den übrigen unbehandelten Körperflächen wachsenden schwarzen Haaren, grau. Es konnten keine wesentlichen Unterschiede bezüglich des Ergrauungsgrades der mit 8-Methoxypsoralen und der mit 5-Methoxypsoralen behandelten Mäuse festgestellt werden, so daß beide Photosensibilisatoren in diesem Modell zur Untersuchung des Ergrauens von Haaren als gleichwertig betrachtet werden.

Mit dem zuvor beschriebenen Modell läßt sich das Ergrauen von Haaren zuverlässig und in einem Zeitraum, der im wesentlichen durch die natürliche Haarwuchsgeschwindigkeit der Versuchstiere bestimmt wird, reproduzieren.

Dieses Modell ist daher geeignet, Wirkstoffe, die das Ergrauen von Haaren verhindern, zu testen.

**Versuch zur Verhinderung des Ergrauens der Haare durch die Verwendung einer superoxiddismutasehaltigen Zubereitung**

Der Rücken von 15 schwarzen Neuseelandmäusen, die bei Versuchsbeginn zwischen 3 und 12 Monaten alt waren, wurde auf einer Fläche von 6 cm x 2,5 cm epiliert.

**A** Auf die rechte Hälfte der epilierten Hautoberfläche der schwarzen Neuseelandmäuse wurden fünfmal im zeitlichen Abstand von jeweils etwa 12 Stunden 80 Mikroliter des folgenden superoxiddismutasehaltigen Gels aufgetragen und kurz einmassiert:

| Gel mit Superoxiddismutase | |
|---|---|
| 0,01 g | Superoxiddismutase aus Rindererythrocyten einer spezifischen Aktivität von 3000 Einheiten/mg Protein |
| 0,10 g | Acrylsäurepolymer |
| 0,03 g | Triethanolamin |
| 99,86 g | Wasser |
| 100,00 g | |

Etwa 40 Minuten nachdem die fünfte Portion des superoxiddismutasehaltigen Gels einmassiert worden war und etwa 48 Stunden nach dem Epilieren, wurden 50 Mikroliter einer alkoholischen Lösung der folgenden Zusammensetzung auf die rechte Hälfte der epilierten Hautfläche der Mäuse aufgetragen und kurz einmassiert:

| | |
|---|---|
| 0,050 g | 8-Methoxypsoralen |
| 69,965 g | Ethanol |
| 29,985 g | Wasser |
| 100,000 g | |

**B** Auf die linke Hälfte der epilierten Hautoberfläche der schwarzen Neuseelandmäuse wurden fünfmal im zeitlichen Abstand von jeweils etwa 12 Stunden 80 Mikroliter des folgenden Gels aufgetragen und kurz einmassiert:

| Gel ohne Superoxiddismutase | |
|---|---|
| 0,10 g | Acrylsäurepolymer |

(fortgesetzt)

| Gel ohne Superoxiddismutase | |
|---|---|
| 0,03 g | Triethanolamin |
| 99,87 g | Wasser |
| $\overline{100,00\ g}$ | |

Etwa 40 Minuten nachdem die fünfte Portion des Gels ohne Superoxiddismutase einmassiert worden war und etwa 48 Stunden nach dem Epilieren, wurden 50 Mikroliter der unter A beschriebenen alkoholischen 8-Methoxypsoralenlösung auf die linke Hälfte der epilierten Hautoberfläche der schwarzen Neuseelandmäuse aufgetragen und kurz einmassiert.

Die so vorbehandelten Mäuse wurden 20 Minuten nach dem Auftragen der alkoholischen 8-Methoxypsoralenlösung 20 Minuten lang bei einer Wellenlänge von 365 nm mit ultraviolettem Licht bestrahlt, wobei der Abstand der Mäuse zur Ultraviolettlampe 20 cm betrug. Für die Bestrahlung wurde die vorstehend beschriebene Black Ray Longwave Ultraviolettlampe in der dort beschriebenen Weise angewendet. Die maximale Strahlungsdosis für die Mäuse betrug 4 J/cm$^2$.

Unmittelbar nach der Bestrahlung wurden nochmals 80 Mikroliter des superoxiddismutasehaltigen Gels auf die rechte und 80 Mikroliter des Gels ohne Superoxiddismutase auf die linke epilierte Hälfte der Hautoberfläche der schwarzen Neuseelandmäuse aufgetragen und einmassiert.

Auf der linken Hälfte der epilierten Hautoberfläche der 15 Versuchstiere, die mit dem superoxiddismutasefreien Gel gemäß Versuchsbeschreibung Teil B behandelt worden waren, wuchsen graue Haare nach.

Auf der mit dem superoxiddismutasehaltigen Gel gemäß Versuchsbeschreibung Teil A behandelten rechten Hälfte der epilierten Hautoberfläche der 15 Versuchstiere wuchsen dieselben schwarzen Haare nach, wie sie sich auf den unbehandelten Körperflächen der Versuchstiere befanden.

Das Versuchsergebnis zeigt, daß das Ergrauen von Haaren durch die erfindungsgemäße Verwendung eines Radikalfängers, wie zum Beispiel Superoxiddismutase, verhindert werden kann.

Sämtliche in der Anmeldung angegebenen Prozentzahlen stellen Gewichtsprozente dar.

**Patentansprüche**

1. Verwendung einer aus Bakterien, Pilzen oder Blut gewonnenen natürlichen oder entsprechenden gentechnologisch hergestellten Superoxiddismutase in einer physiologisch verträglichen, zur Behandlung der Haare und insbesondere der Kopfhaut geeigneten Zubereitung zur Verhinderung oder Verzögerung des Ergrauens menschlicher Haare.

2. Verwendung nach Anspruch 1, **dadurch gekennzeichnet,** daß die Superoxiddismutase in der Zubereitung in einer Menge von 0,01 bis 10 Gewichtsprozent enthalten ist.

3. Verwendung nach den Ansprüchen 1 und 2, **dadurch gekennzeichnet,** daß die Zubereitung nach der Anwendung nicht wieder ausgespült wird und sie die Superoxiddismutase in einer Menge von 0,01 bis 0,5 Gewichtsprozent enthalt.

4. Verwendung nach den Ansprüchen 1 und 2, **dadurch gekennzeichnet,** daß die Zubereitung nach der Anwendung wieder ausgespült wird und sie die Superoxiddismutase in einer Menge von 0,2 bis 1 Gewichtsprozent enthält.

5. Verwendung nach den Ansprüchen 1 bis 4, **dadurch gekennzeichnet,** daß die Superoxiddismutase aus Erythrozyten gewonnen wurde.

6. Verwendung nach den Ansprüchen 1 bis 5, **dadurch gekennzeichnet,** daß die Superoxiddismutase eine spezifische Aktivität von mindestens 3000 bis 3200 Einheiten/mg Protein aufweist.

7. Verwendung nach Anspruch 1, **dadurch gekennzeichnet,** daß die physiologisch verträgliche Zubereitung ein Shampoo, eine Haarspülung, ein Haargel, eine Haarkur oder ein Haarwasser ist.

**Claims**

1. Use of natural superoxide dismutase extracted from bacteria, fungi, blood, or of the corresponding enzyme pro-

duced by genetic engineering techniques, in a Physiologically tolerated preparation for the treatment of the hair and in particular of the scalp in order to prevent or to delay hair graying.

2. Use as defined in claim 1, characterized in that the preparation contains superoxide dismutase at a concentration between 0.01 to 10 grs per cent.

3. Use as defined in claim 1 and 2 further characterized in, that the composition is not rinsed from the hair after the applying and the composition contains superoxyde dismutase in a concentration of 0.01 to 0.5 grs per cent.

4. Use as defined in claims 1 and 2, characterized in that the composition is rinsed after the applying and that it contains superoxide dismutase at a concentration between 0.2 to 1 grs per cent.

5. Use as defined in claims 1 to 4, characterize in that the superoxide dismutase has been extracted from erythrocytes.

6. Use as defined in claims 1 to 5, wherein the superoxide dismutase has a specific activity of at least 3000 to 3200 units/mg protein.

7. Use a defined in claim 1, wherein the physiologically tolerated hair treatment composition is a shampoo, a hair rinse, a hair gel, a hair care agent or a hair tonic.

**Revendications**

1. Utilisation d'une superoxyde dismutase naturelle extraite de bactéries, de levures, du sang, ou de l'enzyme correspondante produite par des techniques de génie génétique, dans une préparation bien supportée sur le plan physiologique pour le traitement des cheveaux et en particulier du corps chevelu, dans le but d'empêcher ou de retarder le grisonement des cheveaux humains.

2. Utilisation selon revendication 1, ainsi caractérisée, que cette préparation contient la superoxyde dismutase á une concentration entre 0.01 et 10 grs pour cent.

3. Utilisation selon les revendications 1 et 2, ainsi caractérisée, que cette préparation n'est pas rincée après application et qu'elle contient la superoxyde dismutase à une concentration entre 0.01 et 0.5 grs pour cent.

4. Utilisation selon les revendications 1 et 2, ainsi caractérisée que cette préparation est rincée après application et qu'elle contient la superoxyde dismutase a une concentration entre 0.2 et 1 grs pour cent.

5. Utilisation selon les revendications 1 à 4, ainsi caractérisée, que la superoxyde dismutase a été extraite à partir d'erythrocytes.

6. Utilisation selon les revendications 1 à 5, ainsi caractérisée, que la superoxyde dismutase a une activité spécifique d'au moins 3000 à 3200 unités/mg protéine.

7. Utilisation selon revendication 1, ainsi caractérisée, que cette préparation, bien supportée sur le plan physiologique, est un shampooing, un produit de rinçage, un gel, une cure ou une lotion capillaire.